# EUROPÄISCHE PATENTANMELDUNG

(11) **EP 2 280 023 A1**
(43) Veröffentlichungstag der Anmeldung: **02.02.2011**
(21) Anmeldenummer: 10009968.8
(22) Anmeldetag: 23.03.2004
(51) Int. Cl.: C07K 7/00, A61K 38/08

(54) **An MHC-Moleküle bindende Tumor-assoziierte Peptide**

(30) Priorität: 24.03.2003 DE 10313819
(62) Teilanmeldung aus: 04722556.0
(71) Anmelder: Immatics Biotechnologies GmbH, 72076 Tübingen (DE)
(72) Erfinder: Rammensee, Hans-Georg, 72070 Tübingen (DE); Stevanovic, Stefan, 72074 Tübingen (DE); Weinschenk, Toni, 73773 Aichwald (DE); Trautwein, Claudia, 42489 Wülfrath (DE); Djengjel, Jörn, 72076 Tübingen (DE); Schoor, Oliver, 72070 Tübingen (DE)
(74) Vertreter: Krauss, Jan

(57) **Zusammenfassung**

Die Erfindung betrifft ein Tumor-assoziiertes Peptid mit einer Aminosäuresequenz, die ausgewählt ist aus der Gruppe bestehend aus SEQ ID-Nr. 1 bis SEQ ID-Nr. 101 aus dem beiliegenden Sequenzprotokoll, wobei das Peptid die Fähigkeit aufweist, an ein Molekül des menschlichen Haupt-Histokompatibilitäts-Komplexes (MHC) Klasse-I zu binden. Die Erfindung betrifft darüber hinaus die Verwendung der Peptide und der für die Peptide kodierenden Nukleinsäuren zur Herstellung eines Arzneimittels und zur Behandlung von Tumorerkrankungen und/oder adenomatöser Erkrankungen. Ferner wird eine pharmazeutische Zusammensetzung beschrieben, die mindestens eines der Peptide aufweist.

## Beschreibung

Die vorliegende Erfindung betrifft Tumor-assoziierte Peptide, die die Fähigkeit aufweisen, an ein Molekül des menschlichen Haupt-Histokompatibilitäts-Komplexes (MHC), Klasse I, zu binden.

Derartige Peptide werden bspw. in der Immuntherapie von Tumorerkrankungen eingesetzt.

Bei der Eliminierung von Tumorzellen durch das Immunsystem spielt die Erkennung von Tumor-assoziierten Antigenen (TAA) durch Komponenten des Immunsystems eine herausragende Rolle. Diesem Mechanismus liegt die Voraussetzung zugrunde, dass zwischen Tumorzellen und normalen Zellen qualitative oder quantitative Unterschiede bestehen. Um eine anti-Tumor-Antwort herbeizuführen, müssen die Tumorzellen Antigene exprimieren, gegen welche eine immunologische Antwort erfolgt, die für die Eliminierung des Tumors ausreicht.

Beteiligt bei der Abstoßung von Tumoren sind insbesondere CD8-exprimierende zytotoxische T-Lymphozyten (im folgenden CTL). Zur Auslösung einer derartigen Immunreaktion durch zytotoxische T-Zellen müssen den T-Zellen dazu fremde Proteine/Peptide präsentiert werden. T-Zellen erkennen Antigene als Peptidfragmente nur dann, wenn diese an Zelloberflächen von MHC-Molekülen präsentiert werden. Diese MHC-Moleküle ("major histocompatibility complex") sind Peptidrezeptoren, die normalerweise Peptide innerhalb der Zelle binden, um sie zu der Zelloberfläche zu transportieren. Dieser Komplex aus Peptid und MHC-Molekül kann durch die T-Zellen erkannt werden. Die MHC-Moleküle des Menschen werden auch als humane Leukozyten-Antigene (HLA) bezeichnet.

Es gibt zwei Klassen von MHC-Molekülen: MHC-Klasse-I-Moleküle, die auf den meisten Zellen mit Kern vorkommen, präsentieren Peptide, die durch proteolytischen Abbau endogener Proteine entstehen. MHC-Klasse-II-Moleküle kommen nur auf professionellen Antigen-präsentierenden Zellen (APC) vor und präsentieren Peptide exogener Proteine, die im Verlauf der Endozytose von APC aufgenommen und verarbeitet werden. Komplexe aus Peptid und MHC-Klasse-I werden von CD8-positiven zytotoxischen T-Lymphozyten erkannt, Komplexe aus Peptid und MHC-Klasse-II werden von CD4-Helfer-T-Zellen erkannt.

Damit ein Peptid eine zelluläre Immunantwort auslösen kann, muss es an ein MHC-Molekül binden. Dieser Vorgang ist vom Allel des MHC-Moleküls und von der Aminosäuresequenz des Peptids abhängig. MHC-Klasse-I-bindende Peptide sind in der Regel 8-10 Reste lang und enthalten in ihrer Sequenz zwei konservierte Reste ("Anker"), die mit der entsprechenden Bindungsfurche des MHC-Moleküls interagieren.

Damit das Immunsystem eine effektive CTL-Antwort gegen Tumorabgeleitete Peptide starten kann, müssen diese Peptide nicht nur in der Lage sein, an die bestimmten MHC-Klasse-I-Moleküle zu binden, die von den Tumorzellen exprimiert werden, sondern sie müssen auch von T-Zellen mit spezifischen T-Zell-Rezeptoren (TCR, "T-cell receptor") erkannt werden.

Das Hauptziel zur Entwicklung einer Tumorvakzine ist die Identifizierung und Charakterisierung von Tumor-assoziierten Antigenen, die durch CD8⁺ CTL erkannt werden.

Die Antigene, die von den Tumor-spezifischen zytotoxischen T-Lymphozyten erkannt werden, bzw. deren Epitope, können Moleküle aus sämtlichen Proteinklassen sein, wie z.B. Enzyme, Rezeptoren, Transkriptionsfaktoren, etc. Eine andere wichtige Klasse Tumor-assoziierter Antigene sind Gewebe-spezifische Strukturen, wie bspw. CT ("cancer testis")-Antigene, die in verschiedenen Tumorarten und in gesundem Hodengewebe exprimiert werden.

Damit die Proteine durch die zytotoxischen T-Lymphozyten als Tumor-spezifisches Antigen erkannt werden, und um somit in einer Therapie eingesetzt werden zu können, müssen bestimmte Voraussetzungen vorliegen: Das Antigen soll hauptsächlich von Tumorzellen exprimiert werden und von Normalgeweben nicht oder nur in geringeren Mengen als in den Tumoren. Weiterhin ist wünschenswert, wenn das betreffende Antigen nicht nur in einer Tumorart, sondern auch in weiteren in hoher Konzentration vorliegt. Unbedingt essentiell ist auch das Vorliegen von Epitopen in der Aminosäuresequenz des Antigens, da solche von einem Tumor-assoziierten Antigen abgeleiteten Peptide ("immunogene Peptide") zu einer T-Zell-Antwort führen sollen, sei es *in vitro* oder *in vivo.*

TAAs stellen somit ein Ausgangspunkt für die Entwicklung einer Tumorvakzine dar. Die Methoden zur Identifizierung und Charakterisierung der TAAs beruhen zum einen auf dem Einsatz von in Patienten bereits induzierten CTL oder basieren auf der Erstellung differentieller Transkriptionsprofile zwischen Tumoren und Normalgeweben.

Das Auffinden von Genen, die in Tumorgeweben überexprimiert sind, oder die in derartigen Geweben selektiv exprimiert werden, liefert jedoch keine präzise Information für einen Einsatz der von diesen Genen transkribierten Antigene in der Immuntherapie. Dies hat die Ursache darin, dass jeweils nur einzelne Epitope dieser Antigene für einen derartigen Einsatz geeignet sind, da nur die Epitope der Antigene - nicht das gesamte Antigen - durch MHC-Präsentierung eine T-Zell-Antwort hervorrufen. Daher ist es wichtig, diejenigen Peptide von überexprimierten oder selektiv exprimierten Proteinen zu selektieren, die mit MHC-Molekülen präsentiert werden, wodurch Angriffspunkte für die spezifische Tumorerkennung durch zytotoxische T-Lymphozyten gewonnen werden könnten.

Vor diesem Hintergrund ist es Aufgabe der vorliegenden Erfindung, mindestens eine neue Aminosäuresequenz für ein derartiges Peptid bereitzustellen, welches die Fähigkeit aufweist, an ein Molekül des menschlichen Haupt-Histokompatibilitäts-Komplexes (MHC)-Klasse-I zu binden.

Diese Aufgabe wird erfindungsgemäß gelöst durch die Bereitstellung eines Tumor-assoziierten Peptids mit einer Aminosäuresequenz, die ausgewählt ist aus der Gruppe bestehend aus SEQ ID-Nr. 1 bis SEQ ID-Nr. 101 aus dem beiliegenden Sequenzprotokoll, wobei das Peptid die Fähigkeit aufweist, an ein Molekül des menschlichen Haupt-Histokompatibilitäts-Komplexes (MHC) Klasse-I zu binden.

Die der Erfindung zugrunde liegende Aufgabe wird auf diese Weise vollkommen gelöst.

Dabei versteht sich, dass die vom Tumor identifizierten Peptide zur Gewinnung größerer Mengen und zum Einsatz für die unten aufgeführten Zwecke synthetisiert oder in Zellen zur Expression gebracht werden.

Die Erfinder konnten die obengenannten Peptide als spezifische Liganden von MHC-Klasse-I-Molekülen aus Tumorgewebe isolieren und identifizieren. Dabei werden hierin mit dem Begriff "Tumorassoziiert" Peptide bezeichnet, die aus Tumormaterial isoliert und identifiziert wurden. Diese Peptide, die auf echten (primären) Tumoren präsentiert werden, unterliegen also der Antigenprozessierung in einer Tumor-Zelle.

Die spezifischen Liganden können in der Krebstherapie eingesetzt werden, z.B. um eine Immunantwort gegen Tumorzellen zu induzieren, die die entsprechenden Antigene exprimieren, von denen die Peptide abstammen.

Eine solche Immunantwort in Form einer Induktion von CTL kann zum einen in vivo erreicht werden. Dazu wird einem Patienten, der an einer mit dem TAA assoziierten Tumorerkrankung leidet, das Peptid bspw. in Form einer pharmazeutischen Zusammensetzung verabreicht.

Andererseits kann eine CTL-Antwort auf einen Tumor, der die Antigene, von denen die Peptide abstammen, exprimiert, auch ex vivo ausgelöst werden. Dazu inkubiert man die CTL-Vorläuferzellen zusammen mit Antigen-präsentierenden Zellen und den Peptiden. Anschließend kultiviert man die dadurch stimulierten CTL und verabreicht diese aktivierten CTL dem Patienten.

Weiterhin besteht die Möglichkeit, APC ex vivo mit den Peptiden zu beladen und diese beladenen APC dem Patienten zu verabreichen, der im Tumorgewebe das Antigen exprimiert, von dem das Peptid abstammt. Die APC können dann wiederum in vivo den CTL das Peptid präsentieren und sie aktivieren.

Die erfindungsgemäßen Peptide können aber auch als diagnostische Reagenzien eingesetzt werden.

So kann mit den Peptiden herausgefunden werden, ob in einer CTL-Population spezifisch gegen ein Peptid gerichtete CTL vorliegen oder durch eine Therapie induziert wurden.

Außerdem kann mit den Peptiden die Zunahme von Vorläufer T-Zellen getestet werden, die eine Reaktivität gegen das definierte Peptid aufweisen.

Ferner kann das Peptid als Marker dazu verwendet werden, um den Krankheitsverlauf eines Tumors zu verfolgen, der das Antigen exprimiert, von dem das Peptid abstammt.

In der beigefügten Tabelle 1 sind die identifizierten Peptide aufgeführt. In der Tabelle sind außerdem die Proteine angegeben, von denen die Peptide abstammen und die jeweilige Positionen der Peptide in den betreffenden Proteinen. Dabei sind die englischen Bezeichnungen der Proteine beibehalten worden, um missverständliche Übersetzungen zu vermeiden. Ferner sind jeweils die Acc-Nummern angegeben, die in der Genbank des "National Center for Biotechnology Information" des National Institute of Health geführt werden (siehe http:\\www.ncbi.nlm.nih.gov).

Die Erfinder konnten die Peptide (oder Liganden) aus Nierenzelltumoren zweier Patienten, RCC68 und RCC44, isolieren.

Aus den Tumoren der Patienten konnten 101 Liganden identifiziert werden, welche an die HLA-Subtypen HLA-A*02, HLA-A*29, HLA-B*15 oder HLA-B*45 (Patient RCC68) und an HLA-A*3201, HLA-A*1101, HLA-B*4002, HLA-B*2705 oder HLA-Cw*0202 (Patient RCC44) gebunden waren.

Einige der Liganden stammten von stark exprimierten sogenannten "Housekeeping" Genen ab, die in den meisten Geweben gleichmäßig exprimiert werden, viele zeichneten sich aber durch gewebespezifische und tumorspezifische Expression aus.

So konnten einige Peptide identifiziert werden, die von Proteinen abstammen, die insbesondere in Tumorgewebe überexprimiert werden. So konnten bspw. Fragmente von Vimentin (ALRDVRQQY, Position 268-276, SEQ ID-Nr. 7; EENFAVEA, Position 348-355, SEQ ID-Nr. 15 ; MEENFAVEA, Position 347-355; NYIDKVRFL, Position 116-124) identifiziert werden. Young et al., Expression profiling of renal epithelial neoplasms: a method for tumor classification and discovery of diagnostic molecular markers, 2001, Am. J. Pathol., 158:1639-1651) zeigten, dass dieses Protein in Gewebe von Nierenzelltumoren überexprimiert war.

Die Erfinder konnten außerdem u.a. Liganden identifizieren, die von Alpha-Catenin, (LQHPDVAAY, Position 229-237, SEQ ID-Nr. 43) und Beta-Catenin (AQNAVRLHY, Position 481-489, SEQ ID-Nr. 8), abstammen.

Ferner konnten die Erfinder in eigenen Versuchen zeigen, dass es unter Verwendung von beispielhaft ausgewählten Peptiden möglich war, *in vitro* zytotoxische T-Lymphozyten (CTL) zu generieren, die jeweils spezifisch für die ausgewählten Peptide waren. Mit diesen CTL konnten gezielt Tumorzellen abgetötet werden, welche die entsprechenden Proteine exprimierten und welche darüber hinaus von verschiedenen Tumor-Zelllinien unterschiedlicher Patienten stammten. Ferner lysierten die genannten CTL auch bspw. dendritische Zellen, die zuvor mit den jeweiligen Peptiden "gepulst" (beladen) wurden. Somit konnte gezeigt werden, dass mit den erfindungsgemäßen Peptiden als Epitope humane T-Zellen *in vitro* aktiviert werden konnten. Die Erfinder konnten demnach nicht nur zeigen, dass CTL, die aus peripheren Blut-mononukleären-Zellen (PBMNC) eines Patienten gewonnen wurden und die für ein bestimmtes Peptid spezifisch waren, Zellen der gleichen Tumorart eines anderen Patienten abtöten konnten. Die Erfinder zeigten außerdem, dass mit diesen CTL auch Zellen anderer Tumorarten lysiert werden konnten.

In einer bevorzugten Ausführungsform können auch Peptide zur Stimulierung einer Immunantwort eingesetzt werden, die die Sequenz ID-Nr. 1 bis'101 aufweisen und bei denen zumindest eine Aminosäure durch eine andere Aminosäure mit ähnlichen chemischen Eigenschaften ersetzt ist.

Dies sind mit Bezug auf die jeweiligen MHC-Subtypen bspw. die Ankeraminosäuren, die durch Aminosäuren mit ähnlichen chemischen Eigenschaften ersetzt werden können. So kann bspw. bei Peptiden, die mit dem MHC-Subtyp HLA-A*02 assoziiert sind, an Position 2 Leucin mit Isoleucin, Valin oder mit Methionin und umgekehrt ausgetauscht werden, und am C-Terminus Leucin mit Valin, Isoleucin und Alanin, die allesamt unpolare Seitenketten aufweisen.

Weiterhin ist es möglich, Peptide mit der Sequenz ID-Nr. 1 bis 101 einzusetzen, die N- oder/und C-terminal zumindest eine weitere Aminosäure aufweisen oder bei denen zumindest eine Aminosäure deletiert ist.

Weiterhin können Peptide mit der Sequenz ID-Nr. 1 bis 101 eingesetzt werden, bei denen zumindest eine Aminosäure chemisch modifiziert ist.

Die variierende(n) Aminosäure(n) ist(sind) dabei so gewählt, dass durch die Variation die Immunogenität des Peptids nicht beeinträchtigt ist, d.h. eine ähnliche Bindungsaffinität an das MHC-Molekül und die Fähigkeit zur T-Zell-Stimulierung aufweist.

Erfindungsgemäß kann das Peptid zur Behandlung von Tumorerkrankungen und/oder adenomatöser Erkrankungen verwendet werden.

Die zu behandelnden Tumorerkrankungen umfassen dabei bspw. Nieren-, Brust-, Pankreas-, Magen-, Hoden- und/oder Hautkrebs. Die Aufzählung der Tumorerkrankungen ist dabei lediglich beispielhaft und soll den Verwendungsbereich nicht eingrenzen. Dass die erfindungsgemäßen Peptide für eine solche Verwendung geeignet sind, konnten die Erfinder in eigenen Versuchen zeigen. Darin wurde nachgewiesen, dass eigens generierte CTL, die spezifisch für bestimmte Peptide waren, effektiv und selektiv Tumorzellen abtöten konnten.

Für einen Einsatz von Tumor-assoziierten Antigenen in einer Tumorvakzine sind grundsätzlich mehrere Applikationsformen möglich. So beschrieben Tighe et al., 1998, Gene vaccination: plasmid DNA is more than just a blueprint, Immunol. Today 19(2):89-97, dass das Antigen entweder als rekombinantes Protein mit geeigneten Adjuvantien bzw. Trägersystemen oder als die für das Antigen kodierende cDNA in Plasmidvektoren verabreicht werden kann. In diesen Fällen muss das Antigen im Körper des Patienten von Antigen-präsentierenden Zellen (APC) verarbeitet und präsentiert werden, damit eine Immunantwort ausgelöst wird.

Melief et al., 1996, Peptide-based cancer vaccines, Curr. Opin. Immunol. 8:651-657, zeigen eine weitere Möglichkeit, nämlich die Verwendung von synthetischen Peptiden als Vakzine.

Das Peptid kann dabei in einer bevorzugten Ausführungsform mit Zugabe von Adjuvantien verwendet werden, oder aber auch in Alleinstellung.

Als Adjuvans kann bspw. der Granulocyte-macrophage-colonystimulating-factor (GM-CSF) eingesetzt werden. Weitere Beispiele für solche Adjuvantien sind Aluminiumhydroxid, Emulsionen von Mineralölen, wie bspw. das Freund'sche Adjuvans, Saponine oder Siliciumverbindungen.

Die Verwendung mit Adjuvantien bietet den Vorteil, dass die durch das Peptid ausgelöste Immunantwort verstärkt werden kann und/oder dass das Peptid stabilisiert wird.

In einer anderen bevorzugten Ausführungsform wird das Peptid gebunden auf einer Antigen-präsentierenden Zelle eingesetzt.

Diese Maßnahme hat den Vorteil, dass die Peptide dem Immunsystem, insbesondere den zytotoxischen T-Lymphozyten (CTL), präsentiert werden können. Dadurch können die CTL die Tumorzellen erkennen und spezifisch abtöten. Als Antigen-präsentierende Zellen sind bspw. dendritische Zellen, Monozyten oder B-Lymphozyten für einen solchen Einsatz geeignet.

Dabei werden die Zellen bspw. *ex vivo* mit den Peptiden beladen werden. Andererseits besteht auch die Möglichkeit, die Zellen mit der für die Peptide kodierenden DNA oder der entsprechenden RNA zu transfizieren, um dann die Peptide auf den Zellen zur Expression zu bringen.

Die Erfinder konnten in eigenen Versuchen zeigen, dass es möglich ist, dendritische Zellen (DC) mit spezifischen Peptiden zu beladen, und dass diese beladenen dendritischen Zellen Peptidspezifische CTL aktivieren. Dies bedeutet, dass das Immunsystem stimuliert werden kann, CTL gegen die Tumore zu entwickeln, welche die entsprechenden Peptide exprimieren.

Die das Peptid tragenden Antigen-präsentierenden Zellen können dabei entweder direkt verwendet werden oder aber vor einem Einsatz bspw. mit dem Hitzeschock-Protein gp96 aktiviert werden. Dieses Hitzeschock-Protein induziert die Expression von MHC-Klasse I-Molekülen und von costimulierenden Molekülen wie B7 und stimuliert außerdem die Produktion von Zytokinen. Dadurch wird insgesamt die Auslösung von Immunantworten gefördert.

In einer anderen bevorzugten Ausführungsform werden die Peptide zur Markierung von Leukozyten, insbesondere von T-Lymphozyten verwendet.

Diese Verwendung ist von Vorteil, wenn mit den Peptiden herausgefunden werden soll, ob in einer CTL-Population spezifisch gegen ein Peptid gerichtete CTL vorliegen.

Ferner kann das Peptid als Marker zur Beurteilung eines Therapieverlaufes bei einer Tumorerkrankung verwendet werden.

Auch bei anderen Immunisierungen oder Therapien kann das Peptid für das Monitoring der Therapie eingesetzt werden. Somit ist das Peptid nicht nur therapeutisch, sondern auch diagnostisch einsetzbar.

In einer weiteren Ausführungsform werden die Peptide zur Herstellung eines Antikörpers eingesetzt.

Polyklonale Antikörper können in herkömmlicher Weise durch Immunisierung von Tieren mittels Injektion der Peptide und anschließender Reinigung des Immunglobulins gewonnen werden.

Monoklonale Antikörper können nach Standardprotokollen hergestellt werden, wie bspw. in Methods Enzymol. (1986), 121, Hybridoma technology and monoclonal antibodies, beschrieben.

Die Erfindung betrifft außerdem in einem weiteren Aspekt eine pharmazeutische Zusammensetzung, die eines oder mehrere der Peptide enthält.

Diese Zusammensetzung dient bspw. der parenteralen Verabreichung, bspw. subkutan, intradermal oder intramuskulär, oder der oralen Verabreichung. Dabei sind die Peptide in einem pharmazeutisch annehmbaren, vorzugsweise wässrigen, Träger gelöst oder suspendiert. Darüber hinaus kann die Zusammensetzung Hilfsstoffe, wie bspw. Puffer, Bindemittel, Verdünnungsmittel, etc. enthalten.

Die Peptide können auch zusammen mit immunstimulierenden Substanzen, z.B. Zytokinen, verabreicht werden. Eine umfassende Darstellung von Hilfsstoffen, wie sie bei einer derartigen Zusammensetzung verwendet werden können, ist bspw. in A. Kibbe, Handbook of Pharmaceutical Excipients, 3. Ed., 2000, American Pharmaceutical Association and pharmaceutical press, dargestellt.

Das Mittel kann dabei zur Prävention, Prophylaxe und/oder Therapie von Tumorerkrankungen und/oder adenomatöser Erkrankungen eingesetzt werden.

Das pharmazeutische Mittel, das zumindest eines der Peptide mit der Sequenz ID-Nr. 1 bis 101 enthält, wird einem Patienten verabreicht, der an einer Tumorerkrankung leidet, mit der das betreffende Peptid bzw. Antigen assoziiert ist. Dadurch kann eine Tumor-spezifische Immunantwort auf Basis Tumorspezifischer CTL ausgelöst werden.

Die in der pharmazeutischen Zusammensetzung vorliegende Menge des Peptids oder der Peptide liegt dabei in einer therapeutisch effektiven Menge vor. Dabei können die in der Zusammensetzung enthaltenen Peptide auch an mindestens zwei verschiedene HLA-Typen binden.

Die vorliegende Erfindung betrifft in einem weiteren Aspekt Nukleinsäuremoleküle, die für die Peptide mit der Sequenz ID-Nr. 1 bis 101 kodieren, sowie die Verwendung von zumindest einem der Nukleinsäuremoleküle zur Herstellung eines Arzneimittels zur Behandlung von Tumorerkrankungen und/oder adenomatöser Erkrankungen.

Die Nukleinsäuremoleküle können dabei DNA- oder RNA-Moleküle sein und ebenfalls für die Immuntherapie von Krebserkrankungen eingesetzt werden. Dabei induziert das von dem Nukleinsäuremolekül exprimierte Peptid eine Immunantwort gegen Tumorzellen, die das Peptid exprimieren.

Erfindungsgemäß können die Nukleinsäuremoleküle auch in einem Vektor vorliegen.

Darüber hinaus betrifft die Erfindung Zellen, die mit Hilfe des Nukleinsäuremoleküls, welches für die Peptide kodiert, genetisch derart verändert wurde, dass sie ein Peptid mit der Sequenz ID-Nr. 1 bis 101 produziert.

Die Zellen werden hierfür mit der für die Peptide kodierenden DNA oder der entsprechenden RNA transfiziert, wodurch die Peptide auf den Zellen zur Expression gebracht werden. Für einen solchen Einsatz sind als Antigen-präsentierende Zellen bspw. dendritische Zellen, Monozyten oder andere humane Zellen geeignet, die für die Ko-Stimulation geeignete Moleküle, wie beispielsweise B7.1 oder B7.2, exprimieren.

Die Erfindung betrifft ferner ein diagnostisches Verfahren, bei dem das Vorhandensein eines der neuen Peptide als diagnostischer Marker verwendet wird, sowie ein Verfahren zur Behandlung eines pathologischen Zustandes, bei dem eine Immunantwort gegen ein interessierendes Protein ausgelöst wird, wobei eine therapeutisch wirksame Menge zumindest eines der neuen Peptide verabreicht wird.

Die Erfinder haben erkannt, dass die neuen Peptide auch als Marker für einen pathologischen Zustand verwendet werden können, so dass ein entsprechendes diagnostisches Verfahren, bei dem eine Blutprobe des Patienten genommen und auf übliche Weise auf das Vorhandensein von gegen eines der neuen Peptide gerichteten Lymphozyten untersucht wird, als Früherkennung oder zur gezielten Auswahl einer geeigneten Behandlung verwendet werden kann.

Ferner betrifft die Erfindung ein elektronisches Speichermedium, das die Aminosäuresequenz zumindest eines der neuen Peptide und/oder die Nukleinsäuresequenz von für die neuen Peptide kodierenden Nukleinsäuremoleküle enthält.

Ausgehend von diesem Speichermedium können dann bei Vorliegen einer entsprechenden Indikation die Informationen für die Peptide schnell bereitgestellt werden, die zur Behandlung des pathologischen Zustandes geeignet sind.

Es versteht sich, dass die vorstehend genannten und die nachstehend noch zu erläuternden Merkmale nicht nur in der jeweils angegebenen Kombination, sondern auch in Alleinstellung verwendbar sind, ohne den Rahmen der vorliegenden Erfindung zu verlassen.

Ausführungsbeispiele der Erfindung werden in den nachfolgenden Beispielen erläutert.

### Beispiel 1

### 1.1. Patientenproben

Von der Abteilung für Urologie, Universität Tübingen, wurden zwei Proben erhalten, die von Patienten stammten, die histologisch bestätigte Nierenzelltumore aufwiesen. Beide Patienten hatten keine präoperative Therapie erhalten. Patient Nr. 1 (im folgenden mit RCC68 bezeichnet) besaß die folgende HLA-Typisierung: HLA-A*02 A*29 B*15 B*45; der Patient Nr. 2 (im folgenden mit RCC44 bezeichnet) HLA-A*3201 A*1101 B*4002 B*2705 Cw*0202.

### 1.2. Isolierung der MHC-Klasse-I-gebundenen Peptide

Die schockgefrorenen Tumorproben wurden prozessiert wie bereits beschrieben in Schirle, M. et al., Identification of tumor-associated MHC class I ligands by a novel T cell-independent approach, 2000, European Journal of Immunology, 30:2216-2225. Die Peptide wurden nach Standardprotokollen isoliert, und zwar unter Verwendung des monoklonalen Antikörpers W6/32, der spezifisch für die HLA-Klasse-I-Moleküle ist, oder des monoklonalen Antikörpers BB7.2, der für HLA-A2 spezifisch ist. Barnstable, C.J. et al., Production of monoclonal antibodies to group A erythrocytes, HLA and other human cell surface antigens-new tools for genetic analysis, 1978, Cell, 14:9-20 und Parham, P. & Brodsky, F.M., Partial purification and some properties of BB7.2. A cytotoxic monoclonal antibody with specificity for HLA-A2 and a variant of HLA-A28, 1981, Hum. Immunol., 3:277-299, beschrieben die Herstellung und Anwendung dieser Antikörper.

### 1.3. Massenspektroskopie

Die Peptidewurden durch "reversed phase HPLC" getrennt (SMART-System, µRPC C2/C18 SC 2.1/19, Amersham Pharmacia Biotech) und die erhaltenen Fraktionen durch nanoESI MS analysiert. Dabei wurde vorgegangen, wie beschrieben in Schirle, M. et al., Identification of tumor-associated MHC class I ligands by a novel T cell-independent approach, 2000, European Journal of Immunology, 30:2216-2225.

Die Peptide, die aus Tumorgewebe gewonnen wurden, wurden wie eben erwähnt durch Kapillar-LC-MS identifiziert, allerdings mit geringfügigen Änderungen: 100 µl der Proben wurden jeweils geladen, entsalzt und auf einer 300 µm * 5 mm C18 µ-Vorsäule (LC Packings) vorkonzentriert. Das Lösungsmittel und die Probe wurden mittels einer Spritzenpumpe (PHD 2000, Harvard Apparatur, Inc.) mit einer abgedichteten 100 µl-Spritze (1710 RNR, Hamilton) mit einer Geschwindigkeit von 2 µl/min zugeführt. Zur Trennung der Peptide wurde die Vorkonzentrierungssäule vor eine 75 µm * 250 mm C-18-Säule (LC Packings) geschaltet. Anschließend wurde ein binärer Gradient mit 25-60% B innerhalb von 70 min gefahren, wobei die Flussrate von 12 µl/min auf ungefähr 300 nl/min reduziert wurde, und zwar unter Verwendung eines TEE-Anschlusses (ZT1C, Valco) und einer 300 µm * 150 mm C-18-Säule.

Um sicherzustellen, dass das System frei von restlichen Peptiden war, wurde jeweils eine Leer-Probe gemessen. Online-Fragmentierung wurde wie beschrieben durchgeführt, und die Spektren der Fragmente wurden manuell analysiert. Die Datenbankrecherchen (NCBInr, EST) wurden unter Verwendung von MASCOT durchgeführt (http://www.matrixscience.com).

### 1.4. Identifizierung der MHC-Klasse-I-Liqanden aus Tumorgewebe der Patienten RCC68 und RCC44

In dem beigefügten Sequenzprotokoll und in der beigefügten Tabelle 1 sind die Liganden aufgeführt, die an die HLA-Moleküle der Patienten RCC68 und TCC44 gebunden waren. Die Peptide, die mit HLA-A*02 assoziiert waren, wiesen das allel-spezifische Peptidmotiv auf: So waren an Position 2 Leucin, Valin, Isoleucin, Alanin oder Methionin und am C-Terminus Leucin, Valin, Isoleucin, oder Alanin zu finden. Die meisten der Liganden stammten von sogenannten "housekeeping"-Proteinen ab, jedoch konnten auch Liganden von Proteinen identifiziert werden, die mit Tumoren assoziiert sind.So konnten bspw. Fragmente von Vimentin (ALRDVRQQY, Position 268-276, SEQ ID-Nr. 7; EENFAVEA, Position 348-355, SEQ ID-Nr. 15 ; MEENFAVEA, Position 347-355; NYIDKVRFL, Position 116-124) identifiziert werden. Young et al., Expression profiling of renal epithelial neoplasms: a method for tumor classification and discovery of diagnostic molecular markers, 2001, Am. J. Pathol., 158:1639-1651) zeigten, dass dieses Protein in Gewebe von Nierenzelltumoren überexprimiert war.

### 1.5. Nachweis von Peptid-spezifischen T-Zellen im normalen CD8⁺-T-Zell-Repertoir

Zum Nachweis von Peptid-spezifischen T-Zellen wurden mononukleäre Zellen aus peripherem Blut gesunder Patienten mit den betreffenden HLA-A*Subtypen-Tetrameren gefärbt, welche mit betreffenden Peptiden konstituiert waren: Zur Herstellung der Tetramere wurden rekombinante HLA-A*Subtypen-Moleküle *in vitro* mit den Peptiden konstituiert, durch Gelfiltration gereinigt, biotinyliert und mit Streptavidin zur Verknüpfung der Monomere gemischt.

Grundsätzlich werden die Ergebnisse der Doppelfärbungen durch Analyse mittels FACS ausgewertet und die spezifische Bindungen der Peptid-Tetramere nachgewiesen.

### Beispiel 2

Um die Präsentation der ausgewählten Peptide durch Tumorzellen und die Erkennung der Peptide durch CTL zu analysieren, wurden *in vitro* CTL induziert, die spezifisch für die ausgewählten Peptide waren. Dazu wurden dendritische Zellen (DC) eingesetzt, die aus peripheren Blut-mononukleären-Zellen (PBMNC) von gesunden Spendern stammten, welche den betreffenden HLA-(Sub)Typ aufwiesen.

### 2.1. Gewinnung der DC

Die DC wurden durch Ficoll/Paque-(Biochrom, Berlin, Deutschland)-Dichtegradienten-Zentrifugation aus PBMNC von heparinisiertem Blut isoliert. Das heparinisierte Blut wurde aus "buffy coat"-Präparationen gesunder Spender der Blutbank der Universität Tübingen gewonnen. Die Zellen wurden auf 6-well-Platten (Falcon, Heidelberg, Deutschland) (1 x 10⁷ Zellen/ 3 ml pro well) in RP10 Medium (RPMI 1640, ergänzt mit 10 % Hitze-inaktiviertem fetalem Kälberserum und mit Antibiotika) ausgesät. Nach einer 2-stündigen Inkubation bei 37°C und 5 % CO₂ wurden die nicht-adhärierenden Zellen entfernt und die adhärierenden Blutmonozyten in RP10 Medium kultiviert, wobei folgende Zytokine ins Medium ergänzend zugegeben wurden: humanes rekombinantes GM-CSF (granulocyte makrophage colony stimulating factor; Leukomax, Novartis; 100ng/ml), Interleukin IL-4 (R&D Systems, Wiesbaden, Deutschland; 1000 IU(ml) und TNF-α (Tumor-Nekrose-Faktor α) (R&D Systems, Wiesbaden, Deutschland; 10 ng/ml).

### 2.2. Synthese der Peptide

Die beispielhaft ausgewählten Peptide wurden durch Verwendung von F-moc (9-Fluorenylmethyloxycarbonyl) -Schutzgruppen auf einem Peptid-Synthetisierer (432A, Applied Biosystems, Weiterstadt, Deutschland) synthetisiert und durch "reversed phase" HPLC und Massenspektroskopie analysiert. Auf diese Weise können ausreichende Mengen an den identifizierten Peptiden hergestellt werden.

### 2.3. Induktion einer Antigen-spezifischen CTL-Antwort unter Einsatz von restringierten synthetischen Peptiden

Zur Induktion von CTL wurden die in Schritt 2.1. gewonnenen DC (5 x 10⁵) mit den aus Schritt 2.2. erhaltenen Peptiden mit je 50 µg/ml für 2 Stunden gepulst, anschließend gewaschen und mit 2,5 x 10⁶ autologen PBMNC in RP10 Medium inkubiert. Nach einer 7-tägigen Kultivierungszeit wurden die Zellen mit autologen, Peptid-gepulsten PBMNC restimuliert. Dabei wurde 1 ng/ml humanes rekombinantes Interleukin IL-2 (R&D Systems) an den Tagen 1, 3 und 5 hinzugefügt. Die zytotoxische Aktivität von auf diese Weise induzierten CTL wurde an Tag 5 nach der letzten Restimulierung mittels eines standardisierten ⁵¹Cr-Freisetzungs-Assays (siehe unten, unter 2.4.: CTL-Assay) untersucht.

### 2.4. CTL-Assay

Für die CTL-Assays wurden als Target-Zellen Tumorzellen, Peptid-gepulste Zellen verschiedener Zellinien und autologe DC verwendet. Peptid-gepulste Zellen wurden mit 50 µg/ml Peptid 2 Stunden lang gepulst. Alle Target-Zellen wurden in RP10Medium (RPMI 1640, ergänzt mit 10 % Hitze-inaktiviertem fetalem Kälberserum und mit Antibiotika) 1 Stunde lang bei 37°C mit [⁵¹Cr]Natriumchromat (⁵¹Cr) markiert. Anschließend wurden jeweils 10⁴ Zellen/pro well auf eine 96-well-Platte mit abgerundetem Boden gegeben. Verschiedene Mengen an CTL wurden hinzugefügt, um ein Endvolumen von 200 µl zu erreichen, mit anschließender Inkubation für 4 Stunden bei 37°C. Danach wurden die Überstände (50µl/well) geerntet und in einem beta-Platten-Zähler ausgezählt. Die spezifische Lyse wurde in Prozent folgendermaßen berechnet: 100 x (experimentelle Freisetzung - spontane Freisetzung / maximale Freisetzung - spontane Freisetzung). Die spontane und die maximale Freisetzung wurde jeweils in Gegenwart von entweder Medium oder 2 % Triton X-100 bestimmt.

### 2.5. Ergebnisse der CTL-Induktion

### a) CTL-zytotoxische Aktivität gegenüber Peptid-gepulsten DC

In ⁵¹Cr-Freisetzungs-Assays (siehe unter 2.4.) wurde die zytotoxische Aktivität induzierter CTL (siehe unter 2.3.) gegenüber T2- oder DC-Zellen getestet. Die T2-Zellinie ist HLA-A*02-positiv und TAP (Transporter associated with Antigen processing) -defizient; (TAP-Peptid-Transporter transportieren Peptid-Fragmente eines Proteinantigens vom Zytosol ins endoplasmatische Retikulum, wo sie mit MHC-Molekülen assoziieren.)

Die Ergebnisse dieser Freisetzungs-Assasy zeigten, dass mit CTL-Zelllinien, die nach 2-wöchiger Restimulation gewonnen wurden, eine Antigen-spezifische Abtötung der Zellen erreicht werden konnte: Nur diejenigen Zellen wurden durch eine ansteigende Menge an CTL abgetötet, die die jeweils ausgewählten Peptide präsentierten; die mit irrelevanten Peptiden beladenen Kontrollzellen wurden nicht abgetötet. Dadurch konnte die Spezifität der zytolytischen Aktivität gezeigt werden.

### b) CTL-zytotoxische Aktivität gegenüber Tumorzelllinien

In einem nächsten Schritt wurde wiederum anhand eines ⁵¹Cr-Freisetzungs-Assays getestet, ob die CTL, die spezifisch für die ausgewählten Peptide waren, Tumorzellen erkennen und lysieren, welche die ausgewählten Peptide endogen exprimieren.

Hierzu wurden verschiedende ⁵¹Cr-markiertedie entsprechenden HLA-Moleküle exprimierenden Zelllinien eingesetzt: HCT 116 (Darmkrebs; erhalten von Prof. G. Pawelec, Tübingen, Deutschland), A 498, MZ 1257 und MZ 1774 (Nierenzellkarzinom; erhalten Prof. A. Knuth, Frankfurt, Deutschland), MCF-7 (Brustkrebs; käuflich erworben von der ATCC, American Type Culture Collection), Mel 1479 (Melanom; erhalten von Prof. G. Pawelec, Tübingen, Deutschland) und U 266 (multiples Myelom; erhalten von Prof. G. Pawelec, Tübingen, Deutschland). Diese Zellinien exprimieren bestimmte Proteine als Zielstrukturen ("targets"). Die B-Zellinie Croft (EBV(Epstein-Barr-Virus)-immortalisiert; HLA-A*02-positiv; erhalten von O.J. Finn, Pittsburgh, USA) und die Zelllinie SK-OV-3 (Ovarialtumor; HLA-A*03-positiv; erhalten von O.J. Finn, Pittsburgh, USA) wurden als Negativkontrollen in die Studien mit aufgenommen. K 562 Zellen (bspw. erhältlich bei der Deutschen Sammlung von Mikroorganismen und Zellkulturen, DSMZ; ACC 10) wurden verwendet, um die Aktivität natürlicher Killerzellen (NK) zu bestimmen, da diese Zelllinie hoch sensitiv gegenüber diesen Killerzellen ist.

Alle Zelllinien wurden in RP10 Medium (RPMI 1640, ergänzt mit 10 % Hitze-inaktiviertem fetalem Kälberserum und mit Antibiotika) kultiviert.

Mit den o.g. Tumorzelllinien und der wie unter 2.3. induzierten CTL wurden ⁵¹Cr-Freisetzungsassays (siehe unter 2.4.) durchgeführt.

Bei diesen Tests lysierten die CTL, die für die ausgewählten Peptide jeweils spezifisch sind, effizient Tumorzellen, die sowohl das entsprechende HLA-Molekül als auch die ausgewählten Peptide exprimieren. Die spezifische Lyse wurde - wie oben unter 2.4. angegeben - durch die ⁵¹Cr-Freisetzung gemessen. Die Kontroll-Zelllinie SK-OV-3 (HLA-A-*02-negativ) hingegen wurde durch die CTL nicht lysiert, die durch die Peptide induziert wurden, welche von HLA-A*02 gebunden waren. Dies zeigte, dass die Peptide im Zusammenhang mit den entsprechenden HLA-Molekülen auf den Tumorzellen präsentiert werden müssen, um die Target-Zellen effizient zu lysieren. Ferner wird dadurch die Antigen-Spezifität und die MHC-Restriktion der CTL bestätigt. Die *in vitro* durch die Peptide induzierten CTL-zellen erkannten darüber hinaus auch nicht die Zelllinie K562, was zeigt, dass die zytotoxische Aktivität nicht durch Natürliche Killerzellen (NK)-zellen vermittelt wurde.

### c) Inhibitions-Assays

Um die Antigen-Spezifität und die MHC-Restriktion der *in-vitro*induzierten CTL weiter zu verifizieren, wurden Inhibitions-Assays mit nicht-⁵¹Cr-markierten ("kalten") Inhibitor-Zelllinien durchgeführt.

Hierbei wurde die Fähigkeit von Peptid-gepulsten Zelllinien analysiert, die Lyse von Tumor-Zellen zu inhibieren, bzw. zu kompetitieren. Dazu wurde ein Überschuss an Inhibitor (also an gepulsten, unmarkierten Zellen) eingesetzt. Das Verhältnis des Inhibitors (Peptid-gepulste Zellen) zum Target (Tumor-Zellen) betrug 20:1. Bei Lyse der Inhibitor-Zelllinien konnte kein ⁵¹Cr freigesetzt werden, da die Inhibitor-Zelllinien unmarkiert waren.

Als Inhibitor wurde die Zelllinie T2 (HLA-A*02; TAP-defizient; siehe unter 2.5.a)) eingesetzt. Diese Zelllinie T2 wurde vor den Assays jeweils mit den relevanten Peptiden oder einem irrelevanten Kontrollpeptid gepulst.

Ohne vorliegen der Inhibitor-Zellen wurde eine Lyse der Tumorzellen durch CTL beobachtet. Ferner konnte gezeigt werden, dass bei einem Überschuss an Inhibitor-Target keine Lyse der Tumorzellen stattfand (und somit keine ⁵¹Cr-Freisetzung), sofern das Inhibitor-Target mit den entsprechenden Peptiden gepulst war.

Die Aktivität der CTL richtete sich auf die im Überschuss vorliegenden, unmarkierten T2-Zellen, so dass diese und nicht die Tumorzellen lysiert wurden. Die T2-Zellen, die mit einem irrelevanten Peptid gepulst waren, konnten die Lyse der Tumorzellen durch die CTL nicht inhibieren, so dass freigesetztes ⁵¹Cr gemessen werden konnte.

Die MHC-Restriktion und die Antigen-Spezifität der cytotoxischen Aktivität, die durch die HLA-A*02-Peptid-induzierten CTL vermittelt wurde, konnte unter Verwendung eines HLA-A*02-spezifischen monoklonalen Antikörpers und in einem Inhibitions-Assay mit unmarkiertem ("kaltem") Inhibitor bestätigt werden: Die A 498-Tumorzellen wurden durch Zugabe des HLA-A*02-spezifischen Antikörpers (monoklonaler Antikörper BB7.2, IgG2b, erhalten von S. Stefanovic, Tübingen) blockiert, so dass sie durch Zugabe der CTL nicht lysiert wurden und kein ⁵¹Cr freigesetzt wurde. Als Kontrolle diente ein unspezifischer Antikörper, der das HLA-A*02-Molekül nicht blockierte (ChromPure Maus IgG, Dianova, Deutschland. Für diese Inhibierungs-Versuche wurden die Zellen vor Aussaat auf den 96-well-Platten 30 min. mit 10 µg/ml Antikörper inkubiert.

Ferner zeigte sich, dass die T2-Kompetitions-Zellinie, die mit einem irrelevanten Peptid gepulst wurde, die CTL-vermittelte Lyse der Tumorzellinie A 498 nicht inhibieren konnte, dass jedoch die mit dem entsprechenden Peptid gepulste T2-Inhibitor-Zellinie die Lyse der Tumor-Zelllinie inhibieren konnte, so dass in letzterem Fall keine ⁵¹Cr-Freisetzung gemessen werden konnte.

### d) Spezifische Lyse transfizierter DC

In einem nächsten Versuch wurde die zytotoxische Aktivität der CTL bei einem autologen Versuchsansatz analysiert. Hierzu wurden autologe DC, die aus den gleichen PBMNC gewonnen wurden wie diejenigen, die für die CTL-Induktion (siehe unter 2.2.) verwendet wurden, als Target-Zellen eingesetzt. Vor Durchführung des CTL-Assays wurden die DC mit RNA elektroporiert, welche zuvor entweder aus Tumor-Zelllinien isoliert wurde oder welche Kontroll-RNA darstellte. Die Gesamt-RNA wurde aus den Tumorzellen mittels des QIAGEN Rneasy Mini Kits (QIAGEN, Hilden, Deutschland) nach Angaben des Herstellers isoliert. Menge und Reinheit der RNA wurde photometrisch bestimmt und in Aliquots bei -80°C aufbewahrt.

Vor der Elektroporation an Tag 6 wurden unreife DC zweimal mit Serum-freiem X-VIVO 20 Medium (BioWhittaker, Walkersville, USA) gewaschen und in einer Endkonzentration von 2 x 10⁷ Zellen/ml resuspendiert. Anschließend wurden 200 µl der Zellsuspension mit 10 µg der Gesamt-RNA gemischt und in einer 4 mm Küvette mittels eines Easyject Plus™ (Peqlab, Erlangen Deutschland) elektroporiert (Parameter: 300 V, 150 µF, 1540 Ω, Pulszeit: 231 ms). Nach der Elektroporation wurden die Zellen sofort in RP10 Medium überführt und wieder in den Inkubator gegeben. Mehr als 80 % der Zellen waren nach der Elektroporation lebensfähig.

Nach Durchführung des CTL-Assays mit durch die ausgewählten Peptide induzierten CTL (siehe unter 2.4.) konnte eine spezifische Lyse von DC beobachtet werden, welche mit RNA von Peptidexprimierenden Tumor-Zelllinien elektroporiert waren. DC, die

**Tabelle 1**

| | **Sequenz** | **Position/Genart** | **Acc. Nr.** | **SEQ ID-Nr.** |
|---|---|---|---|---|
| 1. | AAFPGASLY | 63-71 DAZ associated protein 2 | NM_014764 | SEQ ID-Nr. 1 |
| 2. | AELATRALP | 137-145 junction plakoglobin | NM_002230 | SEQ ID-Nr. 2 |
| 3. | AFFAERLYY | 397-405 annexin A7 | NM_001156 | SEQ ID-Nr. 3 |
| 4. | ALATLIHQV | 26-34 COP9 constitutive photomorphogenic homolog subunit 7A (Arabidopsis) | NM_016319 | SEQ ID-Nr. 4 |
| 5. | ALAVIITSY | 318-326 ATPase, H+ transporting, lysosomal (vacuolar proton pump) membrane sector associated protein M8-9 | NM_005765 | SEQ ID-Nr. 5 |
| 6. | ALQEMVHQV | 806-814 enhancer of filamentation 1 | NM_006403 | SEQ ID-Nr. 6 |
| 7. | ALRDVRQQY | 268-276 vimentin | NM_003380 | SEQ ID-Nr. 7 |
| 8. | AQNAVRLHY | 481-489 catenin (cadherin-associated protein), beta 1, 88kDa | NM_001904 | SEQ ID-Nr. 8 |
| 9. | AQPGFFDRF | 1006-1014 collagen, type VI, alpha 2 (COL6A2), transcript variant 2C2 | NM_001849 | SEQ ID-Nr. 9 |
| 10. | AVCEVALDY | 2260-2268 spectrin, beta, non-erythrocytic 1 | NM_003128 | SEQ ID-Nr. 10 |
| 11. | AVLGAVVAV | 161-169 Cwl antigen | M12679 | SEQ ID-Nr. 11 |
| 12. | DAILEELSA | 154-162 hypothetical protein FLJ11749 | NM_024591 | SEQ ID-Nr. 12 |
| 13. | EEHPTLLTEA | 101-110 actin, alpha 2, smooth muscle, aorta | NM_001613 | SEQ ID-Nr. 13 |
| 14. | EEMPQVHTP | 715-723 MCM3 minichromosome maintenance deficient 3 (S. cerevisiae) | NM_002388 | SEQ ID-Nr. 14 |
| 15. | EENFAVEA | 348-355 vimentin | NM_003380 | SEQ ID-Nr. 15 |
| 16. | EENKLIYTP | 56-64 binder of Arl Two | NM_012106 | SEQ ID-Nr. 16 |
| 17. | FAEGFVRAL | 110-118 v-jun sarcoma virus 17 oncogene homolog (avian) | NM_002228 | SEQ ID-Nr. 17 |
| 18. | FFGETSHNY | 235-243 matrin 3 | NM_018834 | SEQ ID-Nr. 18 |
| 19. | FLPHMAYTY | 931-939 zinc finger homeobox 1b | NM_014795 | SEQ ID-Nr. 19 |
| 20. | GEPRFISVGY | 42-51 major histocompatibility com- plex, class I, C | Z46810 | SEQ ID-Nr. 20 |
| 21. | GLATDVQTV | 55-63 proteasome (prosome, macropain) subunit, beta type, 3 | NM_002795 | SEQ ID-Nr. 21 |
| 22. | GLNDETYGY | 161-169 ATPase, Na+/K+ transporting, beta 1 polypeptide | NM_001677 | SEQ ID-Nr. 22 |
| 23. | GQEFIRVGY | 103-111 anti-silencing function 1B | NM_018154 | SEQ ID-Nr. 23 |
| 24. | GQFPGHNEF | 76-84 CDC42 effector protein (Rho GTPase binding) 3 | NM_006449 | SEQ ID-Nr. 24 |
| 25. | GQPWVSVTV | 121-129 FLJ00063 | AC005912 | SEQ ID-Nr. 25 |
| 26. | GYLHDFLKY | 254-262 mortality factor 4 like 2 | NM_012286 | SEQ ID-Nr. 26 |
| 27. | HQITVLHVY | 137-145 homolog of yeast long chain polyunsaturated fatty acid elongation enzyme 2 | NM_021814 | SEQ ID-Nr. 27 |
| 28. | HVIDVKFLY | 163-171 damage-specific DNA binding protein 1, 127kDa | NM_001923 | SEQ ID-Nr. 28 |
| 29. | HVNDLFLQY | 484-492 KIAA1005 | AB023222 | SEQ ID-Nr. 29 |
| 30. | IAMATVTAL | 249-257 aldolase A, fructose-bisphosphate | NM_000034 | SEQ ID-Nr. 30 |
| 31. | IGIDLGTTY | 7-15 heat shock 70kDa protein 1A | NM_005345 | SEQ ID-Nr. 31 |
| 32. | ILHDDEVTV | 15-23 ribosomal protein, large, P1 | NM_001003 | SEQ ID-Nr. 32 |
| 33. | IQKESTLHL | 61-69 ubiquitin A-52 residue ribosomal protein fusion product 1 | NM_003333 | SEQ ID-Nr. 33 |
| 34. | ISRELYEY | 70-77 clone MGC:39264 IMAGE:5087938 | BC022821 | SEQ ID-Nr. 34 |
| 35. | KLHGVNINV | 59-67 RNA binding motif protein 4 | NM_002896 | SEQ ID-Nr. 35 |
| 36. | KQMEQVAQF | 89-97 transgelin | NM_003186 | SEQ ID-Nr. 36 |
| 37. | KVADMALHY | 296-304 chaperonin containing TCP1, subunit 8 (theta) | NM_006585 | SEQ ID-Nr. 37 |
| 38. | LEEDSAREI | 68-76 LOC204689 | XM_119113 | SEQ ID-Nr. 38 |
| 39. | LLAERDLYL | 576-584 transglutaminase 2 (C polypeptide, protein-glutamine-gamma-glutamyltransferase) | NM_004613 | SEQ ID-Nr. 39 |
| 40. | LLDEEISRV | 44-52 RNA binding protein HQK-7 | AB067800 | SEQ ID-Nr. 40 |
| 41. | LLYPTEITV | 830-838 integrin, alpha 3 (antigen CD49C, alpha 3 subunit of VLA-3 receptor) | NM_002204 | SEQ ID-Nr. 41 |
| 42. | LMDHTIPEV | 290-298 syndecan binding protein | NM_005625 | SEQ ID-Nr. 42 |
| 43. | LQBPDVAAY | 229-237 catenin (cadherin-associated protein), alpha 1, 102kDa | NM_001903 | SEQ ID-Nr. 43 |
| 44. | MEDIKILIA | 632-640 hypoxia-inducible factor 1, alpha subunit (basic helix-loop-helix transcription factor) | NM_001530 | SEQ ID-Nr. 44 |
| 45. | MEENFAVEA | 347-355 vimentin | NM_003380 | SEQ ID-Nr. 45 |
| 46. | MQKEITAL | 313-320 actin, beta | NM_001101 | SEQ ID-Nr. 46 |
| 47. | NEDLRSWTA | 151-159 HLA-G histocompatibility antigen, class I, G | NM_002127 | SEQ ID-Nr. 47 |
| 48. | NEIKDSVVA | 673-681 eukaryotic translation elongation factor 2 | NM_001961 | SEQ ID-Nr. 48 |
| 49. | NVTQVRAFY | 439-447 catalase | NM_001752 | SEQ ID-Nr. 49 |
| 50. | NYIDKVRFL | 116-124 vimentin | NM_003380 | SEQ ID-Nr. 50 |
| 51. | PTQELGLPAY | 392-401 seryl-tRNA synthetase 2 | NM_017827 | SEQ ID-Nr. 51 |
| 52. | QEQSFVIRA | 422-430 integrin, beta 2 (antigen CD18 (p95), lymphocyte function-associated antigen 1; macrophage antigen 1 (mac-1) beta subunit) | NM_000211 | SEQ ID-Nr. 52 |
| 53. | QQKLSRLQY | 636-644 integrin, alpha 3 (antigen CD49C, alpha 3 subunit of VLA-3 receptor) | NM_002204 | SEQ ID-Nr. 53 |
| 54. | QVAEIVSKY | 217-225 integrin, alpha V (vitronectin receptor, alpha polypeptide, antigen CD51) | NM_002210 | SEQ ID-Nr. 54 |
| 55. | REHAPFLVA | 30-38 transport-secretion protein 2.2 | XM_208570 | SEQ ID-Nr. 55 |
| 56. | RLAAAAAQSVY | 5-15 glutathione peroxidase 1 | NM_000581 | SEQ ID-Nr. 56 |
| 57. | RLASYLDKV | 90-98 keratin 19 | Y00503 | SEQ ID-Nr. 57 |
| 58. | RNADVFLKY | 1020-1028 triple functional domain (PTPRF interacting) | NM_007118 | SEQ ID-Nr. 58 |
| 59. | RQGFVPAAY | 1012-1020 spectrin, alpha, non-erythrocytic 1 (alpha-fodrin) | NM_003127 | SEQ ID-Nr. 59 |
| 60. | RVIEEAKTAF | 198-207 heme oxygenase (decycling) 1 | NM_002133 | SEQ ID-Nr. 60 |
| 61. | RVQPKVTVY | 89-97 MHC class II antigen | AF450316 | SEQ ID-Nr. 61 |
| 62. | RVYPEVTVY | 123-131 MHC HLA-DRB1*0411 | L42143 | SEQ ID-Nr. 62 |
| 63. | SDHHIYL | 218-224 aldolase A, fructose-bisphosphate | NM_000034 | SEQ ID-Nr. 63 |
| 64. | SHAILEALA | 204-212 F-box and leucine-rich repeat protein 8 | NM_018378 | SEQ ID-Nr. 64 |
| 65. | SISGVTAAY | 728-736 IQ motif containing GTPase activating protein 1 | NM_003870 | SEQ ID-Nr. 65 |
| 66. | SPVYVGRV | 216-223 transglutaminase 2 (C polypeptide, protein-glutamine-gamma-glutamyltransferase) | NM_004613 | SEQ ID-Nr. 66 |
| 67. | SQFGTVTRF | 66-74 MKI67 (FHA domain) interacting nucleolar phosphoprotein | NM_032390 | SEQ ID-Nr. 67 |
| 68. | SWNNHSYLY | 156-164 gamma-glutamyl carboxylase | NM_000821 | SEQ ID-Nr. 68 |
| 69. | TFMDHVLRY | 700-708 ATP citrate lyase | NM_001096 | SEQ ID-Nr. 69 |
| 70. | TLADLVHHV | 378-386 transformation/transcription domain-associated protein | NM_003496 | SEQ ID-Nr. 70 |
| 71. | TLGALTVIDV | 1336-1345 hypothetical protein DKFZp434N074 | NM_017539 | SEQ ID-Nr. 71 |
| 72. | TQMPDPKTF | 46-54 HSPC038 protein | NM_016096 | SEQ ID-Nr. 72 |
| 73. | VEHPSLTSP | 170-178 HLA-DR beta gene, exon 2 | M15374 | SEQ ID-Nr. 73 |
| 74. | VEPDHFKVA | 204-212 lectin, galactoside-binding, soluble, 3 (galectin 3) | NM_002306 | SEQ ID-Nr. 74 |
| 75. | VEREVEQV | 64-71 EST reading frame +2 | AI278671 | SEQ ID-Nr. 75 |
| 76. | VFIGTGATGATLY | 20-32 NADH dehydrogenase (ubiquinone) 1 alpha subcomplex, 4, 9kDa | NM_002489 | SEQ ID-Nr. 76 |
| 77. | VLREIAEEY | 822-830 high density lipoprotein binding protein (vigilin) | NM_005336 | SEQ ID-Nr. 77 |
| 78. | VLSLLSSVAL | 27-36 LOC153339 | XM_098362 | SEQ ID-Nr. 78 |
| 79. | VLYDRVLKY | 484-492 signal recognition particle 68kDa | NM_014230 | SEQ ID-Nr. 79 |
| 80. | VMDSKIVQV | 432-440 karyopherin alpha 6 (importin alpha 7) | NM_012316 | SEQ ID-Nr. 80 |
| 81. | VQRTLMAL | 126-133 transgelin | NM_003186 | SEQ ID-Nr. 81 |
| 82. | YFEYIEENKY | 238-247 heterogeneous nuclear ribonucleoprotein U (scaffold attachment factor A) | NM_004501 | SEQ ID-Nr. 82 |
| 83. | YIFKERESF | 303-311 CGI-18 protein | NM_015947 | SEQ ID-Nr. 83 |
| 84. | YVYEYPSRY | 164-172 enhancer of filamentation 1 | NM_006403 | SEQ ID-Nr. 84 |
| 85. | YYRYPTGESY | 354-363 6-phosphofructo-2-kinase/fructose-2,6-biphosphatase 3 | NM_004566 | SEQ ID-Nr. 85 |
| 86. | YYSNKAYQY | 230-238 human immune associated nucleotide 2 | NM_024711 | SEQ ID-Nr. 86 |
| 87. | SSLPTQLFK | 5-13 insulin-like growth factor 1 | NM_000618 | SEQ ID-Nr. 87 |

## Patentansprüche

1. Tumor-assoziiertes Peptid mit der Aminosäuresequenz der SEQ ID No. 17 (Phe Ala Glu Gly Phe Val Arg Ala Leu), wobei das Peptid die Fähigkeit aufweist, an ein Molekül des menschlichen Haupt-Histokompatibilitäts-Komplexes (MHC) Klasse-I zu binden.

2. Peptid nach Anspruch 1, **dadurch gekennzeichnet, dass** N- oder/und C-terminal eine weitere Aminosäure vorhanden ist.

3. Peptid nach Anspruch 1 oder 2, **dadurch gekennzeichnet, dass** zumindest eine Aminosäure deletiert ist und/oder dass zumindest eine Aminosäure chemisch modifiziert ist.

4. Verwendung des Peptids nach einem der Ansprüche 1 bis 3 zur Herstellung eines Arzneimittels zur Behandlung von Tumorerkrankungen und/oder adenomatöser Erkrankungen.

5. Verwendung des Peptids nach einem der Ansprüche 1 bis 3 zur Herstellung einer pharmazeutischen Zusammensetzung zur Behandlung von Tumorerkrankungen und/oder adenomatöser Erkrankungen.

6. Verwendung nach Anspruch 4 oder 5, **dadurch gekennzeichnet, dass** die Erkrankung Nieren-, Brust-, Pankreas-, Magen-, Blasen- und/oder Hodenkrebs ist.

7. Verwendung nach Anspruch 5 oder 6, **dadurch gekennzeichnet, dass** das Peptid zusammen mit einem Adjuvans eingesetzt wird, oder dass das Peptid gebunden auf einer Antigen-präsentierenden Zelle eingesetzt wird.

8. Verwendung des Peptids nach einem der Ansprüche 1 bis 3 zur in vitro-Markierung von Leukozyten, insbesondere von T-Lymphozyten.

9. Verwendung nach Anspruch 8 zur Beurteilung eines Therapieverlaufs bei einer Tumorerkrankung.

10. Verwendung des Peptids nach einem der Ansprüche 1 bis 3 zur Herstellung eines Antikörpers.

11. Pharmazeutische Zusammensetzung enthaltend das Peptid nach einem der Ansprüche 1 bis 3.

12. Nukleinsäuremolekül, kodierend für das Peptid nach einem der Ansprüche 1 bis 3.

13. Vektor umfassend das Nukleinsäuremolekül nach Anspruch 12.

14. Zelle, die mit Hilfe des Nukleinsäuremoleküls nach Anspruch 12 oder mit Hilfe des Vektors nach Anspruch 13 genetisch derart verändert wurde, dass sie in vitro ein Peptid nach einem der Ansprüche 1 bis 3 produziert, wobei die Zelle keine humane embryonale Stammzelle ist.

15. Diagnostisches Verfahren, bei dem das Vorhandensein eines Peptides nach einem der Ansprüche 1 bis 3 als diagnostischer Marker verwendet wird.


SEQUENZPROTOKOLL

<110>  Immatics Biotechnologies GmbH

<120>  An MHC-Moleküle bindende Tumor-assoziierte Peptide

<130>  4648P104

<160>  101

<170>  PatentIn version 3.1

<210>  1
<211>  9
<212>  PRT
<213>  Homo sapiens

<400>  1

Ala Ala Phe Pro Gly Ala Ser Leu Tyr
1               5


<210>  2
<211>  9
<212>  PRT
<213>  Homo sapiens

<400>  2

Ala Glu Leu Ala Thr Arg Ala Leu Pro
1               5


<210>  3
<211>  9
<212>  PRT
<213>  Homo sapiens

<400>  3

Ala Phe Phe Ala Glu Arg Leu Tyr Tyr
1               5


<210>  4
<211>  9
<212>  PRT
<213>  Homo sapiens

<400>  4

Ala Leu Ala Thr Leu Ile His Gln Val
1               5

<210> 5
<211> 9
<212> PRT
<213> Homo sapiens

<400> 5

Ala Leu Ala Val Ile Ile Thr Ser Tyr
1               5


<210> 6
<211> 9
<212> PRT
<213> Homo sapiens

<400> 6

Ala Leu Gln Glu Met Val His Gln Val
1               5


<210> 7
<211> 9
<212> PRT
<213> Homo sapiens

<400> 7

Ala Leu Arg Asp Val Arg Gln Gln Tyr
1               5


<210> 8
<211> 9
<212> PRT
<213> Homo sapiens

<400> 8

Ala Gln Asn Ala Val Arg Leu His Tyr
1               5


<210> 9
<211> 9
<212> PRT
<213> Homo sapiens

<400> 9

Ala Gln Pro Gly Phe Phe Asp Arg Phe
1               5

<210> 10
<211> 9
<212> PRT
<213> Homo sapiens

<400> 10

Ala Val Cys Glu Val Ala Leu Asp Tyr
1               5


<210> 11
<211> 9
<212> PRT
<213> Homo sapiens

<400> 11

Ala Val Leu Gly Ala Val Val Ala Val
1               5


<210> 12
<211> 10
<212> PRT
<213> Homo sapiens

<400> 12

Glu Glu His Pro Thr Leu Leu Thr Glu Ala
1               5                   10


<210> 13
<211> 10
<212> PRT
<213> Homo sapiens

<400> 13

Glu Glu His Pro Thr Leu Leu Thr Glu Ala
1               5                   10


<210> 14
<211> 9
<212> PRT
<213> Homo sapiens

<400> 14

Glu Glu Met Pro Gln Val His Thr Pro
1               5

<210>  15
<211>  8
<212>  PRT
<213>  Homo sapiens

<400>  15

Glu Glu Asn Phe Ala Val Glu Ala
1               5


<210>  16
<211>  9
<212>  PRT
<213>  Homo sapiens

<400>  16

Glu Glu Asn Lys Leu Ile Tyr Thr Pro
1               5


<210>  17
<211>  9
<212>  PRT
<213>  Homo sapiens

<400>  17

Phe Ala Glu Gly Phe Val Arg Ala Leu
1               5


<210>  18
<211>  9
<212>  PRT
<213>  Homo sapiens

<400>  18

Phe Phe Gly Glu Thr Ser His Asn Tyr
1               5


<210>  19
<211>  9
<212>  PRT
<213>  Homo sapiens

<400>  19

Phe Leu Pro His Met Ala Tyr Thr Tyr
1               5

<210> 20
<211> 10
<212> PRT
<213> Homo sapiens

<400> 20

Gly Glu Pro Arg Phe Ile Ser Val Gly Tyr
1               5                   10


<210> 21
<211> 9
<212> PRT
<213> Homo sapiens

<400> 21

Gly Leu Ala Thr Asp Val Gln Thr Val
1               5


<210> 22
<211> 9
<212> PRT
<213> Homo sapiens

<400> 22

Gly Leu Asn Asp Glu Thr Tyr Gly Tyr
1               5


<210> 23
<211> 9
<212> PRT
<213> Homo sapiens

<400> 23

Gly Gln Glu Phe Ile Arg Val Gly Tyr
1               5


<210> 24
<211> 9
<212> PRT
<213> Homo sapiens

<400> 24

Gly Gln Phe Pro Gly His Asn Glu Phe
1               5

<210>  25
<211>  9
<212>  PRT
<213>  Homo sapiens

<400>  25

Gly Gln Pro Trp Val Ser Val Thr Val
1               5


<210>  26
<211>  9
<212>  PRT
<213>  Homo sapiens

<400>  26

Gly Tyr Leu His Asp Phe Leu Lys Tyr
1               5


<210>  27
<211>  9
<212>  PRT
<213>  Homo sapiens

<400>  27

His Gln Ile Thr Val Leu His Val Tyr
1               5


<210>  28
<211>  9
<212>  PRT
<213>  Homo sapiens

<400>  28

His Val Ile Asp Val Lys Phe Leu Tyr
1               5


<210>  29
<211>  9
<212>  PRT
<213>  Homo sapiens

<400>  29

His Val Asn Asp Leu Phe Leu Gln Tyr
1               5

<210>  30
<211>  9
<212>  PRT
<213>  Homo sapiens

<400>  30

Ile Ala Met Ala Thr Val Thr Ala Leu
1               5


<210>  31
<211>  9
<212>  PRT
<213>  Homo sapiens

<400>  31

Ile Gly Ile Asp Leu Gly Thr Thr Tyr
1               5


<210>  32
<211>  9
<212>  PRT
<213>  Homo sapiens

<400>  32

Ile Leu His Asp Asp Glu Val Thr Val
1               5


<210>  33
<211>  9
<212>  PRT
<213>  Homo sapiens

<400>  33

Ile Gln Lys Glu Ser Thr Leu His Leu
1               5


<210>  34
<211>  8
<212>  PRT
<213>  Homo sapiens

<400>  34

Ile Ser Arg Glu Leu Tyr Glu Tyr
1               5

<210>  35
<211>  9
<212>  PRT
<213>  Homo sapiens

<400>  35

Lys Leu His Gly Val Asn Ile Asn Val
1               5


<210>  36
<211>  9
<212>  PRT
<213>  Homo sapiens

<400>  36

Lys Gln Met Glu Gln Val Ala Gln Phe
1               5


<210>  37
<211>  9
<212>  PRT
<213>  Homo sapiens

<400>  37

Lys Val Ala Asp Met Ala Leu His Tyr
1               5


<210>  38
<211>  9
<212>  PRT
<213>  Homo sapiens

<400>  38

Leu Glu Glu Asp Ser Ala Arg Glu Ile
1               5


<210>  39
<211>  9
<212>  PRT
<213>  Homo sapiens

<400>  39

Leu Leu Ala Glu Arg Asp Leu Tyr Leu
1               5

<210> 40
<211> 9
<212> PRT
<213> Homo sapiens

<400> 40

Leu Leu Asp Glu Glu Ile Ser Arg Val
1               5


<210> 41
<211> 9
<212> PRT
<213> Homo sapiens

<400> 41

Leu Leu Tyr Pro Thr Glu Ile Thr Val
1               5


<210> 42
<211> 9
<212> PRT
<213> Homo sapiens

<400> 42

Leu Met Asp His Thr Ile Pro Glu Val
1               5


<210> 43
<211> 9
<212> PRT
<213> Homo sapiens

<400> 43

Leu Gln His Pro Asp Val Ala Ala Tyr
1               5


<210> 44
<211> 9
<212> PRT
<213> Homo sapiens

<400> 44

Met Glu Asp Ile Lys Ile Leu Ile Ala
1               5

<210> 45
<211> 9
<212> PRT
<213> Homo sapiens

<400> 45

Met Glu Glu Asn Phe Ala Val Glu Ala
1               5


<210> 46
<211> 8
<212> PRT
<213> Homo sapiens

<400> 46

Met Gln Lys Glu Ile Thr Ala Leu
1               5


<210> 47
<211> 9
<212> PRT
<213> Homo sapiens

<400> 47

Asn Glu Asp Leu Arg Ser Trp Thr Ala
1               5


<210> 48
<211> 9
<212> PRT
<213> Homo sapiens

<400> 48

Asn Glu Ile Lys Asp Ser Val Val Ala
1               5


<210> 49
<211> 9
<212> PRT
<213> Homo sapiens

<400> 49

Asn Val Thr Gln Val Arg Ala Phe Tyr
1               5

<210>  50
<211>  9
<212>  PRT
<213>  Homo sapiens

<400>  50

Asn Tyr Ile Asp Lys Val Arg Phe Leu
1               5


<210>  51
<211>  10
<212>  PRT
<213>  Homo sapiens

<400>  51

Pro Thr Gln Glu Leu Gly Leu Pro Ala Tyr
1               5                   10


<210>  52
<211>  9
<212>  PRT
<213>  Homo sapiens

<400>  52

Gln Glu Gln Ser Phe Val Ile Arg Ala
1               5


<210>  53
<211>  9
<212>  PRT
<213>  Homo sapiens

<400>  53

Gln Gln Lys Leu Ser Arg Leu Gln Tyr
1               5


<210>  54
<211>  9
<212>  PRT
<213>  Homo sapiens

<400>  54

Gln Val Ala Glu Ile Val Ser Lys Tyr
1               5

<210> 55
<211> 9
<212> PRT
<213> Homo sapiens

<400> 55

Arg Glu His Ala Pro Phe Leu Val Ala
1               5


<210> 56
<211> 11
<212> PRT
<213> Homo sapiens

<400> 56

Arg Leu Ala Ala Ala Ala Ala Gln Ser Val Tyr
1               5                   10


<210> 57
<211> 9
<212> PRT
<213> Homo sapiens

<400> 57

Arg Leu Ala Ser Tyr Leu Asp Lys Val
1               5


<210> 58
<211> 9
<212> PRT
<213> Homo sapiens

<400> 58

Arg Asn Ala Asp Val Phe Leu Lys Tyr
1               5


<210> 59
<211> 9
<212> PRT
<213> Homo sapiens

<400> 59

Arg Gln Gly Phe Val Pro Ala Ala Tyr
1               5

<210> 60
<211> 10
<212> PRT
<213> Homo sapiens

<400> 60

Arg Val Ile Glu Glu Ala Lys Thr Ala Phe
1               5                   10


<210> 61
<211> 9
<212> PRT
<213> Homo sapiens

<400> 61

Arg Val Gln Pro Lys Val Thr Val Tyr
1               5


<210> 62
<211> 9
<212> PRT
<213> Homo sapiens

<400> 62

Arg Val Tyr Pro Glu Val Thr Val Tyr
1               5


<210> 63
<211> 7
<212> PRT
<213> Homo sapiens

<400> 63

Ser Asp His His Ile Tyr Leu
1               5


<210> 64
<211> 9
<212> PRT
<213> Homo sapiens

<400> 64

Ser His Ala Ile Leu Glu Ala Leu Ala
1               5

<210>  65
<211>  9
<212>  PRT
<213>  Homo sapiens

<400>  65

Ser Ile Ser Gly Val Thr Ala Ala Tyr
1               5


<210>  66
<211>  8
<212>  PRT
<213>  Homo sapiens

<400>  66

Ser Pro Val Tyr Val Gly Arg Val
1               5


<210>  67
<211>  9
<212>  PRT
<213>  Homo sapiens

<400>  67

Ser Trp Asn Asn His Ser Tyr Leu Tyr
1               5


<210>  68
<211>  9
<212>  PRT
<213>  Homo sapiens

<400>  68

Ser Trp Asn Asn His Ser Tyr Leu Tyr
1               5


<210>  69
<211>  9
<212>  PRT
<213>  Homo sapiens

<400>  69

Thr Phe Met Asp His Val Leu Arg Tyr
1               5

<210> 70
<211> 9
<212> PRT
<213> Homo sapiens

<400> 70

Thr Leu Ala Asp Leu Val His His Val
1               5


<210> 71
<211> 10
<212> PRT
<213> Homo sapiens

<400> 71

Thr Leu Gly Ala Leu Thr Val Ile Asp Val
1               5                   10


<210> 72
<211> 9
<212> PRT
<213> Homo sapiens

<400> 72

Thr Gln Met Pro Asp Pro Lys Thr Phe
1               5


<210> 73
<211> 9
<212> PRT
<213> Homo sapiens

<400> 73

Val Glu His Pro Ser Leu Thr Ser Pro
1               5


<210> 74
<211> 9
<212> PRT
<213> Homo sapiens

<400> 74

Val Glu Pro Asp His Phe Lys Val Ala
1               5

<210>  75
<211>  8
<212>  PRT
<213>  Homo sapiens

<400>  75

Val Glu Arg Glu Val Glu Gln Val
1               5


<210>  76
<211>  13
<212>  PRT
<213>  Homo sapiens

<400>  76

Val Phe Ile Gly Thr Gly Ala Thr Gly Ala Thr Leu Tyr
1               5                   10


<210>  77
<211>  9
<212>  PRT
<213>  Homo sapiens

<400>  77

Val Leu Arg Glu Ile Ala Glu Glu Tyr
1               5


<210>  78
<211>  10
<212>  PRT
<213>  Homo sapiens

<400>  78

Val Leu Ser Leu Leu Ser Ser Val Ala Leu
1               5                   10


<210>  79
<211>  9
<212>  PRT
<213>  Homo sapiens

<400>  79

Val Leu Tyr Asp Arg Val Leu Lys Tyr
1               5

<210> 80
<211> 9
<212> PRT
<213> Homo sapiens

<400> 80

Val Met Asp Ser Lys Ile Val Gln Val
1               5


<210> 81
<211> 8
<212> PRT
<213> Homo sapiens

<400> 81

Val Gln Arg Thr Leu Met Ala Leu
1               5


<210> 82
<211> 10
<212> PRT
<213> Homo sapiens

<400> 82

Tyr Phe Glu Tyr Ile Glu Glu Asn Lys Tyr
1               5                   10


<210> 83
<211> 9
<212> PRT
<213> Homo sapiens

<400> 83

Tyr Ile Phe Lys Glu Arg Glu Ser Phe
1               5


<210> 84
<211> 9
<212> PRT
<213> Homo sapiens

<400> 84

Tyr Val Tyr Glu Tyr Pro Ser Arg Tyr
1               5

<210>  85
<211>  10
<212>  PRT
<213>  Homo sapiens

<400>  85

Tyr Tyr Arg Tyr Pro Thr Gly Glu Ser Tyr
1               5                   10


<210>  86
<211>  9
<212>  PRT
<213>  Homo sapiens

<400>  86

Tyr Tyr Ser Asn Lys Ala Tyr Gln Tyr
1               5


<210>  87
<211>  9
<212>  PRT
<213>  Homo sapiens

<400>  87

Ser Ser Leu Pro Thr Gln Leu Phe Lys
1               5


<210>  88
<211>  9
<212>  PRT
<213>  Homo sapiens

<400>  88

Ala Thr Phe Pro Asp Thr Leu Thr Tyr
1               5


<210>  89
<211>  10
<212>  PRT
<213>  Homo sapiens

<400>  89

Ser Ile Phe Asp Gly Arg Val Val Ala Lys
1               5                   10

<210> 90
<211> 9
<212> PRT
<213> Homo sapiens

<400> 90

Phe Arg Phe Glu Asn Val Asn Gly Tyr
1               5


<210> 91
<211> 10
<212> PRT
<213> Homo sapiens

<400> 91

Gln Arg Tyr Gly Phe Ser Ala Val Gly Phe
1               5                   10


<210> 92
<211> 10
<212> PRT
<213> Homo sapiens

<400> 92

Ala Arg Leu Ser Leu Thr Tyr Glu Arg Leu
1               5                   10


<210> 93
<211> 9
<212> PRT
<213> Homo sapiens

<400> 93

Gly Arg Tyr Gln Val Ser Trp Ser Leu
1               5


<210> 94
<211> 9
<212> PRT
<213> Homo sapiens

<400> 94

Lys Arg Phe Asp Asp Lys Tyr Thr Leu
1               5

<210>  95
<211>  9
<212>  PRT
<213>  Homo sapiens

<400>  95

Thr Arg Trp Asn Lys Ile Val Leu Lys
1               5


<210>  96
<211>  9
<212>  PRT
<213>  Homo sapiens

<400>  96

Leu Arg Phe Asp Gly Ala Leu Asn Val
1               5


<210>  97
<211>  9
<212>  PRT
<213>  Homo sapiens

<400>  97

Ala Arg Phe Ser Gly Asn Leu Leu Val
1               5


<210>  98
<211>  9
<212>  PRT
<213>  Homo sapiens

<400>  98

Asn Arg Ile Lys Phe Val Ile Lys Arg
1               5


<210>  99
<211>  9
<212>  PRT
<213>  Homo sapiens

<400>  99

Gly Arg Val Phe Ile Ile Lys Ser Tyr
1               5

SEQUENZPROTOKOLL

<110>  Immatics Biotechnologies GmbH

<120>  An MHC-Moleküle bindende Tumor-assoziierte Peptide

<130>  FB15878/A

<160>  101

<170>  PatentIn version 3.1

<210>  1
<211>  9
<212>  PRT
<213>  Homo sapiens

<400>  1

Ala Ala Phe Pro Gly Ala Ser Leu Tyr
1               5


<210>  2
<211>  9
<212>  PRT
<213>  Homo sapiens

<400>  2

Ala Glu Leu Ala Thr Arg Ala Leu Pro
1               5


<210>  3
<211>  9
<212>  PRT
<213>  Homo sapiens

<400>  3

Ala Phe Phe Ala Glu Arg Leu Tyr Tyr
1               5


<210>  4
<211>  9
<212>  PRT
<213>  Homo sapiens

<400>  4

Ala Leu Ala Thr Leu Ile His Gln Val
1               5

<210>  5
<211>  9
<212>  PRT
<213>  Homo sapiens

<400>  5

Ala Leu Ala Val Ile Ile Thr Ser Tyr
1               5


<210>  6
<211>  9
<212>  PRT
<213>  Homo sapiens

<400>  6

Ala Leu Gln Glu Met Val His Gln Val
1               5


<210>  7
<211>  9
<212>  PRT
<213>  Homo sapiens

<400>  7

Ala Leu Arg Asp Val Arg Gln Gln Tyr
1               5


<210>  8
<211>  9
<212>  PRT
<213>  Homo sapiens

<400>  8

Ala Gln Asn Ala Val Arg Leu His Tyr
1               5


<210>  9
<211>  9
<212>  PRT
<213>  Homo sapiens

<400>  9

Ala Gln Pro Gly Phe Phe Asp Arg Phe
1               5

<210>  10
<211>  9
<212>  PRT
<213>  Homo sapiens

<400>  10

Ala Val Cys Glu Val Ala Leu Asp Tyr
1               5


<210>  11
<211>  9
<212>  PRT
<213>  Homo sapiens

<400>  11

Ala Val Leu Gly Ala Val Val Ala Val
1               5


<210>  12
<211>  10
<212>  PRT
<213>  Homo sapiens

<400>  12

Glu Glu His Pro Thr Leu Leu Thr Glu Ala
1               5                   10


<210>  13
<211>  10
<212>  PRT
<213>  Homo sapiens

<400>  13

Glu Glu His Pro Thr Leu Leu Thr Glu Ala
1               5                   10


<210>  14
<211>  9
<212>  PRT

<213>  Homo sapiens

<400>  14

Glu Glu Met Pro Gln Val His Thr Pro
1               5

<210>  15
<211>  8
<212>  PRT
<213>  Homo sapiens

<400>  15

Glu Glu Asn Phe Ala Val Glu Ala
1               5

<210>  16
<211>  9
<212>  PRT
<213>  Homo sapiens

<400>  16

Glu Glu Asn Lys Leu Ile Tyr Thr Pro
1               5

<210>  17
<211>  9
<212>  PRT
<213>  Homo sapiens

<400>  17

Phe Ala Glu Gly Phe Val Arg Ala Leu
1               5

<210>  18
<211>  9
<212>  PRT
<213>  Homo sapiens

<400>  18

Phe Phe Gly Glu Thr Ser His Asn Tyr
1               5

<210>  19

<211>  9
<212>  PRT
<213>  Homo sapiens

<400>  19

Phe Leu Pro His Met Ala Tyr Thr Tyr
1               5

<210>  20
<211>  10
<212>  PRT
<213>  Homo sapiens

<400>  20

Gly Glu Pro Arg Phe Ile Ser Val Gly Tyr
1               5                   10


<210>  21
<211>  9
<212>  PRT
<213>  Homo sapiens

<400>  21

Gly Leu Ala Thr Asp Val Gln Thr Val
1               5


<210>  22
<211>  9
<212>  PRT
<213>  Homo sapiens

<400>  22

Gly Leu Asn Asp Glu Thr Tyr Gly Tyr
1               5


<210>  23
<211>  9
<212>  PRT
<213>  Homo sapiens

<400>  23

Gly Gln Glu Phe Ile Arg Val Gly Tyr
1               5

<210>  24
<211>  9
<212>  PRT
<213>  Homo sapiens

<400>  24

Gly Gln Phe Pro Gly His Asn Glu Phe
1               5

<210>  25
<211>  9
<212>  PRT
<213>  Homo sapiens

<400>  25

Gly Gln Pro Trp Val Ser Val Thr Val
1               5

<210>  26
<211>  9
<212>  PRT
<213>  Homo sapiens

<400>  26

Gly Tyr Leu His Asp Phe Leu Lys Tyr
1               5

<210>  27
<211>  9
<212>  PRT
<213>  Homo sapiens

<400>  27

His Gln Ile Thr Val Leu His Val Tyr
1               5

<210>  28
<211>  9
<212>  PRT
<213>  Homo sapiens

<400>  28

His Val Ile Asp Val Lys Phe Leu Tyr

1                   5


<210>  29
<211>  9
<212>  PRT
<213>  Homo sapiens

<400>  29

His Val Asn Asp Leu Phe Leu Gln Tyr
1               5

<210>  30
<211>  9
<212>  PRT
<213>  Homo sapiens

<400>  30

Ile Ala Met Ala Thr Val Thr Ala Leu
1               5


<210>  31
<211>  9
<212>  PRT
<213>  Homo sapiens

<400>  31

Ile Gly Ile Asp Leu Gly Thr Thr Tyr
1               5


<210>  32
<211>  9
<212>  PRT
<213>  Homo sapiens

<400>  32

Ile Leu His Asp Asp Glu Val Thr Val
1               5


<210>  33
<211>  9
<212>  PRT
<213>  Homo sapiens

<400>  33

Ile Gln Lys Glu Ser Thr Leu His Leu
1               5

<210>  34
<211>  8
<212>  PRT
<213>  Homo sapiens

<400>  34

Ile Ser Arg Glu Leu Tyr Glu Tyr
1               5

<210>  35
<211>  9
<212>  PRT
<213>  Homo sapiens

<400>  35

Lys Leu His Gly Val Asn Ile Asn Val
1               5

<210>  36
<211>  9
<212>  PRT
<213>  Homo sapiens

<400>  36

Lys Gln Met Glu Gln Val Ala Gln Phe
1               5

<210>  37
<211>  9
<212>  PRT
<213>  Homo sapiens

<400>  37

Lys Val Ala Asp Met Ala Leu His Tyr
1               5

<210>  38
<211>  9
<212>  PRT
<213>  Homo sapiens

<400>  38

Leu Glu Glu Asp Ser Ala Arg Glu Ile
1               5


<210>  39
<211>  9
<212>  PRT
<213>  Homo sapiens

<400>  39

Leu Leu Ala Glu Arg Asp Leu Tyr Leu
1               5

<210>  40
<211>  9
<212>  PRT
<213>  Homo sapiens

<400>  40

Leu Leu Asp Glu Glu Ile Ser Arg Val
1               5


<210>  41
<211>  9
<212>  PRT
<213>  Homo sapiens

<400>  41

Leu Leu Tyr Pro Thr Glu Ile Thr Val
1               5


<210>  42
<211>  9
<212>  PRT
<213>  Homo sapiens

<400>  42

Leu Met Asp His Thr Ile Pro Glu Val
1               5


<210>  43
<211>  9

<212>  PRT
<213>  Homo sapiens

<400>  43

Leu Gln His Pro Asp Val Ala Ala Tyr
1               5


<210>  44
<211>  9
<212>  PRT
<213>  Homo sapiens

<400>  44

Met Glu Asp Ile Lys Ile Leu Ile Ala
1               5

<210>  45
<211>  9
<212>  PRT
<213>  Homo sapiens

<400>  45

Met Glu Glu Asn Phe Ala Val Glu Ala
1               5


<210>  46
<211>  8
<212>  PRT
<213>  Homo sapiens

<400>  46

Met Gln Lys Glu Ile Thr Ala Leu
1               5


<210>  47
<211>  9
<212>  PRT
<213>  Homo sapiens

<400>  47

Asn Glu Asp Leu Arg Ser Trp Thr Ala
1               5

<210>  48
<211>  9
<212>  PRT
<213>  Homo sapiens

<400>  48

Asn Glu Ile Lys Asp Ser Val Val Ala
1               5


<210>  49
<211>  9
<212>  PRT
<213>  Homo sapiens

<400>  49

Asn Val Thr Gln Val Arg Ala Phe Tyr
1               5

<210>  50
<211>  9
<212>  PRT
<213>  Homo sapiens

<400>  50

Asn Tyr Ile Asp Lys Val Arg Phe Leu
1               5


<210>  51
<211>  10
<212>  PRT
<213>  Homo sapiens

<400>  51

Pro Thr Gln Glu Leu Gly Leu Pro Ala Tyr
1               5                   10


<210>  52
<211>  9
<212>  PRT
<213>  Homo sapiens

<400>  52

Gln Glu Gln Ser Phe Val Ile Arg Ala
1               5

<210>  53
<211>  9
<212>  PRT
<213>  Homo sapiens

<400>  53

Gln Gln Lys Leu Ser Arg Leu Gln Tyr
1               5


<210>  54
<211>  9
<212>  PRT
<213>  Homo sapiens

<400>  54

Gln Val Ala Glu Ile Val Ser Lys Tyr
1               5

<210>  55
<211>  9
<212>  PRT
<213>  Homo sapiens

<400>  55

Arg Glu His Ala Pro Phe Leu Val Ala
1               5


<210>  56
<211>  11
<212>  PRT
<213>  Homo sapiens

<400>  56

Arg Leu Ala Ala Ala Ala Ala Gln Ser Val Tyr
1               5                   10


<210>  57
<211>  9
<212>  PRT
<213>  Homo sapiens

<400>  57

Arg Leu Ala Ser Tyr Leu Asp Lys Val
1               5

<210>  58
<211>  9
<212>  PRT
<213>  Homo sapiens

<400>  58

Arg Asn Ala Asp Val Phe Leu Lys Tyr
1               5

<210>  59
<211>  9
<212>  PRT
<213>  Homo sapiens

<400>  59

Arg Gln Gly Phe Val Pro Ala Ala Tyr
1               5

<210>  60
<211>  10
<212>  PRT
<213>  Homo sapiens

<400>  60

Arg Val Ile Glu Glu Ala Lys Thr Ala Phe
1               5                   10

<210>  61
<211>  9
<212>  PRT
<213>  Homo sapiens

<400>  61

Arg Val Gln Pro Lys Val Thr Val Tyr
1               5

<210>  62
<211>  9
<212>  PRT
<213>  Homo sapiens

<400>  62

Arg Val Tyr Pro Glu Val Thr Val Tyr
1               5


<210>  63
<211>  7
<212>  PRT
<213>  Homo sapiens

<400>  63

Ser Asp His His Ile Tyr Leu
1               5


<210>  64
<211>  9
<212>  PRT
<213>  Homo sapiens

<400>  64

Ser His Ala Ile Leu Glu Ala Leu Ala
1               5

<210>  65
<211>  9
<212>  PRT
<213>  Homo sapiens

<400>  65

Ser Ile Ser Gly Val Thr Ala Ala Tyr
1               5


<210>  66
<211>  8
<212>  PRT
<213>  Homo sapiens

<400>  66

Ser Pro Val Tyr Val Gly Arg Val
1               5


<210>  67
<211>  9
<212>  PRT

<213>  Homo sapiens

<400>  67

Ser Trp Asn Asn His Ser Tyr Leu Tyr
1               5


<210>  68
<211>  9
<212>  PRT
<213>  Homo sapiens

<400>  68

Ser Trp Asn Asn His Ser Tyr Leu Tyr
1               5


<210>  69
<211>  9
<212>  PRT
<213>  Homo sapiens

<400>  69

Thr Phe Met Asp His Val Leu Arg Tyr
1               5

<210>  70
<211>  9
<212>  PRT
<213>  Homo sapiens

<400>  70

Thr Leu Ala Asp Leu Val His His Val
1               5


<210>  71
<211>  10
<212>  PRT
<213>  Homo sapiens

<400>  71

Thr Leu Gly Ala Leu Thr Val Ile Asp Val
1               5                   10


<210>  72

<211>  9
<212>  PRT
<213>  Homo sapiens

<400>  72

Thr Gln Met Pro Asp Pro Lys Thr Phe
1               5


<210>  73
<211>  9
<212>  PRT
<213>  Homo sapiens

<400>  73

Val Glu His Pro Ser Leu Thr Ser Pro
1               5


<210>  74
<211>  9
<212>  PRT
<213>  Homo sapiens

<400>  74

Val Glu Pro Asp His Phe Lys Val Ala
1               5

<210>  75
<211>  8
<212>  PRT
<213>  Homo sapiens

<400>  75

Val Glu Arg Glu Val Glu Gln Val
1               5


<210>  76
<211>  13
<212>  PRT
<213>  Homo sapiens

<400>  76

Val Phe Ile Gly Thr Gly Ala Thr Gly Ala Thr Leu Tyr
1               5                   10

<210>  77
<211>  9
<212>  PRT
<213>  Homo sapiens

<400>  77

Val Leu Arg Glu Ile Ala Glu Glu Tyr
1               5


<210>  78
<211>  10
<212>  PRT
<213>  Homo sapiens

<400>  78

Val Leu Ser Leu Leu Ser Ser Val Ala Leu
1               5                   10


<210>  79
<211>  9
<212>  PRT
<213>  Homo sapiens

<400>  79

Val Leu Tyr Asp Arg Val Leu Lys Tyr
1               5

<210>  80
<211>  9
<212>  PRT
<213>  Homo sapiens

<400>  80

Val Met Asp Ser Lys Ile Val Gln Val
1               5


<210>  81
<211>  8
<212>  PRT
<213>  Homo sapiens

<400>  81

Val Gln Arg Thr Leu Met Ala Leu

1               5


<210>  82
<211>  10
<212>  PRT
<213>  Homo sapiens

<400>  82

Tyr Phe Glu Tyr Ile Glu Glu Asn Lys Tyr
1               5                   10


<210>  83
<211>  9
<212>  PRT
<213>  Homo sapiens

<400>  83

Tyr Ile Phe Lys Glu Arg Glu Ser Phe
1               5


<210>  84
<211>  9
<212>  PRT
<213>  Homo sapiens

<400>  84

Tyr Val Tyr Glu Tyr Pro Ser Arg Tyr
1               5

<210>  85
<211>  10
<212>  PRT
<213>  Homo sapiens

<400>  85

Tyr Tyr Arg Tyr Pro Thr Gly Glu Ser Tyr
1               5                   10


<210>  86
<211>  9
<212>  PRT
<213>  Homo sapiens

<400>  86

Tyr Tyr Ser Asn Lys Ala Tyr Gln Tyr
1               5

<210>  87
<211>  9
<212>  PRT
<213>  Homo sapiens

<400>  87

Ser Ser Leu Pro Thr Gln Leu Phe Lys
1               5

<210>  88
<211>  9
<212>  PRT
<213>  Homo sapiens

<400>  88

Ala Thr Phe Pro Asp Thr Leu Thr Tyr
1               5

<210>  89
<211>  10
<212>  PRT
<213>  Homo sapiens

<400>  89

Ser Ile Phe Asp Gly Arg Val Val Ala Lys
1               5                   10

<210>  90
<211>  9
<212>  PRT
<213>  Homo sapiens

<400>  90

Phe Arg Phe Glu Asn Val Asn Gly Tyr
1               5

<210>  91
<211>  10
<212>  PRT
<213>  Homo sapiens

<400>  91

Gln Arg Tyr Gly Phe Ser Ala Val Gly Phe
1               5                   10


<210>  92
<211>  10
<212>  PRT
<213>  Homo sapiens

<400>  92

Ala Arg Leu Ser Leu Thr Tyr Glu Arg Leu
1               5                   10


<210>  93
<211>  9
<212>  PRT
<213>  Homo sapiens

<400>  93

Gly Arg Tyr Gln Val Ser Trp Ser Leu
1               5


<210>  94
<211>  9
<212>  PRT
<213>  Homo sapiens

<400>  94

Lys Arg Phe Asp Asp Lys Tyr Thr Leu
1               5

<210>  95
<211>  9
<212>  PRT
<213>  Homo sapiens

<400>  95

Thr Arg Trp Asn Lys Ile Val Leu Lys
1               5


<210>  96
<211>  9

<212>  PRT
<213>  Homo sapiens

<400>  96

Leu Arg Phe Asp Gly Ala Leu Asn Val
1               5


<210>  97
<211>  9
<212>  PRT
<213>  Homo sapiens

<400>  97

Ala Arg Phe Ser Gly Asn Leu Leu Val
1               5


<210>  98
<211>  9
<212>  PRT
<213>  Homo sapiens

<400>  98

Asn Arg Ile Lys Phe Val Ile Lys Arg
1               5


<210>  99
<211>  9
<212>  PRT
<213>  Homo sapiens

<400>  99

Gly Arg Val Phe Ile Ile Lys Ser Tyr
1               5

<210>  100
<211>  9
<212>  PRT
<213>  Homo sapiens

<400>  100

Ser Arg Phe Gly Asn Ala Phe His Leu
1               5

<210> 101
<211> 9
<212> PRT
<213> Homo sapiens

<400> 101

Gly Arg Thr Gly Gly Ser Trp Phe Lys
1               5